# EUROPEAN PATENT APPLICATION

(11) **EP 4 674 349 A1**
(43) Date of publication of application: **07.01.2026**
(21) Application number: 24186034.5
(22) Date of filing: 02.07.2024
(51) Int. Cl.: A61B 6/00, G06T 7/00

(54) **METHOD FOR ESTIMATING A SPLIT RENAL FUNCTION FOR A TRANSFER RENAL FUNCTION OF A TRACER IN THE KIDNEYS**

(71) Applicant: Advanced Neural Nuclear Imaging S.à r.l.-S, 2557 Luxembourg (LU)
(72) Inventor: HUBAUT, Marc-Antoine, 59500 Douai (FR); DUQUESNE, Quentin, 2560 Luxembourg (LU)
(74) Representative: Plasseraud IP

(57) **Abstract**

The present document relates to a method implemented by computer means for estimating, on a dynamic renal scintigraphy of both kidneys of a patient, a split renal function for a transfer renal function of a tracer in the kidneys, said dynamic renal scintigraphy comprising a series of images taken over a global time sequence. The method's automated approach to estimating split renal function on dynamic renal scintigraphy streamlines the process by reducing the need for manual intervention and specialized training. This not only saves time but also minimizes the potential for human error in the calculation process. The computer-implemented steps provide a standardized and reproducible method for calculating the split renal function, in order to assess the relative contribution of each kidney to the overall renal function in a patient.

## Description

### Technical Field

This disclosure relates to a method implemented by computer means for estimating, on a dynamic renal scintigraphy of both kidneys of a patient, a split renal function for a transfer renal function of a tracer in the kidneys.

### Background Art

A dynamic renal scintigraphy, also known as a renal nuclear scan, is a medical imaging technique used to assess the structure and function of the kidneys. It involves the use of a radioactive tracer that is injected into the patient's bloodstream. This tracer is taken up by the kidneys and allows healthcare providers to create images and gather functional information about how the kidneys are working.

Dynamic acquisition of kidneys is performed with a gamma-camera which produce a series of images at a defined rate, showing the repartition of the tracer in the field of view in function of time. Nowadays, two main tracers are recommended to perform a dynamic renal scintigraphy: 99mTc-mercaptoacetyltriglycine (MAG3), a tubular agent, and 99mTc-DTPA, a glomerular agent.

Images are acquired with patient in supine position with camera usually positioned under the table for acquisition in posterior view. In some case (horseshoe or ectopic kidney for example) anterior view is also performed in order to calculate a geometric mean of anterior and posterior view.

A diuretic injection could be performed or not. This injection can be performed before, at the time, or after tracer injection. A vascular phase acquisition can be performed during the firsts one or two minutes after tracer injection, constituted of images acquired during few seconds (usually 1 second per image). Then a dynamic acquisition of 15 or 20-seconds images is usually performed during 20 to 40 min. One or several late static images of few minutes (usually 5 minutes) could be performed after the dynamic acquisition.

Various parameters could be derived from renal scintigraphy. Among them, separated kidney function (SKF) is a parameter that can be accurately calculated in vivo only by performing a renal scintigraphy. Different computation methods could be used to calculate SKF.

Patlak-Rutland method is a bi-compartmental model first described in 1983 (Patlak CS, Blasberg RG, Fenstermacher JD. Graphical Evaluation of Blood-to-Brain Transfer Constants from Multiple-Time Uptake Data. Journal of Cerebral Blood Flow & Metabolism. 1983;3(1):1-7. doi:1 0.1038/jcbfm.1983.1). This method allows a robust and reproducible SKF calculation on dynamic renal scintigraphy. SKF is the relative percentage of total renal function which is assumed by each kidney. The studied function could be glomerular function (with used of DTPA) or tubular function (using a tubular agent like MAG3).

Patlak method may be used to calculate SKF on renal scintigraphy, as it is for another method called area under curve. However, such method could be difficult to use in clinical routine, especially on infant with renal function alteration with high vascular background activity.

Moreover, an accurate computation with this method has to be ensured by a correct definition of kidney regions and vascular pool (heart or aorta) and a correct definition of the time interval in which the calculation is performed. This operation needs specific training and is time-consuming.

### Summary

This disclosure improves the situation by proposing a method able to implement such Patlak method more accurately.

To this aim, it is proposed a method implemented by computer means for estimating, on a dynamic renal scintigraphy of both kidneys of a patient, a split renal function for a transfer renal function of a tracer in the kidneys, said dynamic renal scintigraphy comprising a series of images taken over a global time sequence, said method comprising the following steps:
(a) obtaining said series of images during a global time sequence, at least one image comprising both kidneys,
(b) obtaining a starting time of a parenchymal phase of the global time sequence from said dynamic renal scintigraphy,
(c) defining a vascular region of interest on said images of the global time sequence,
(d) calculating a tracer activity in the vascular region of interest on said images, called vascular tracer activity;
wherein, for each kidney, the method comprises:
(e) obtaining a local time sequence of a plurality of images comprising the kidney from said series of images obtained during the global time sequence,
(f) defining a kidney region of interest on said images of the local time sequence,
(g) calculating a tracer activity in the kidney region of interest for each image of said local time sequence, called renal tracer activity,
(h) determining an end time of the renal activity in the kidney region of interest,
wherein the method further comprises the following step:
(i) estimating a ratio between transfer function coefficients Kf of each kidney at least from the renal and vascular activities in the kidney regions of interest after said starting time;
(j) estimating the split renal function at least from said ratio between the transfer function coefficients.

A tracer refers to an intravenous radioactive substance used to study the uptake, transit, and excretion of the tracer through the kidneys. The tracer allows for the visualization of renal function and anatomy by emitting gamma rays that are detected by a gamma-camera, producing a series of images over time.

The vascular phase refers to the initial stage of the imaging process where the focus is on assessing the blood flow to the kidneys. This phase occurs immediately after the injection of a radioactive tracer into the bloodstream. During this early stage, the tracer is rapidly transported through the renal arteries into the kidneys. Imaging during this phase captures how efficiently blood is delivered to the kidneys.

The parenchymal phase refers to a stage of the imaging process where the radioactive tracer has been absorbed by the renal parenchyma, which is the functional tissue of the kidneys primarily made up of nephrons that perform the essential tasks of blood filtration and urine production. This phase follows the initial vascular phase.

The starting time of a parenchymal phase may be given in the metadata given by the dynamic renal scintigraphy.

A time sequence refers to a series of images acquired at defined intervals over a period, capturing the distribution and movement of the tracer through the kidneys over time. This sequence is used to analyze the uptake, transit, and excretion of the tracer.

The tracer activity refers to the behavior and distribution of the radioactive tracer within the kidneys over time, as detected by a gamma-camera for example. The tracer, such as 99mTc-mercaptoacetyltriglycine (MAG3) or 99mTc-DTPA, is intravenously injected and its uptake, transit, and excretion are imaged to study renal function. The activity is quantified by measuring the intensity of gamma rays emitted from the tracer within the region of interest, which corresponds to the kidneys in this case.

By obtaining a global time sequence of images and defining specific regions of interest for both the vascular area and the kidneys, the method enables the calculation of tracer activity within these regions over time. This allows for the precise estimation of the radio of the transfer function coefficients Kf for each kidney, in order to determine the split renal function. The method's ability to accurately determine the end time of renal activity ensures that the calculation of said ratio is based on the correct interval, thereby improving the reliability of the split renal function estimation.

The method's automated approach to estimating split renal function on dynamic renal scintigraphy streamlines the process by reducing the need for manual intervention and specialized training. This not only saves time but also minimizes the potential for human error in the calculation process. The computer-implemented steps provide a standardized and reproducible method for calculating the split renal function, in order to assess the relative contribution of each kidney to the overall renal function in a patient.

The step (c) may comprise the following substeps:
(c.i) calculating a preliminary average image of images of said global time sequence before the starting time, said average image being the first image of said global time sequence if none before,
(c.ii) identifying on said preliminary average image an upper area with the highest intensity,
(c.iii) defining said vascular region of interest on said upper area.

The vascular region is the area where a spike of activity occurs at the start of the acquisition, as the tracer's vascular bolus is usually in the heart or aorta. A renal scintigraphy may or may not include a dedicated vascular acquisition (vascular phase). In the first case, there are two phases. If there is a vascular phase, it is the first one, followed by the parenchymal phase. If not, the parenchymal phase is the only one visible by the acquisition.

If there is a vascular phase, all images of the phase are summed up to calculate one average image. If there is no vascular phase, the first image of the parenchymal phase is selected as the average image.

An average image refers to a composite image created by averaging the pixel intensities across a series of images. This process is used to enhance the signal-to-noise ratio and provide a clearer representation of the renal activity by reducing the impact of transient vascular background activity.

By calculating a preliminary average image of images of the global time sequence before the starting time, the method provides a basis for identifying the vascular region of interest with enhanced accuracy. This preliminary average image serves to stabilize the representation of the vascular activity, mitigating the impact of transient fluctuations in individual images and thus facilitating a more reliable identification of the vascular region.

Identifying an upper area with the highest intensity on the preliminary average image allows for the precise localization of the vascular region of interest, as heart is above the kidney in a vertical axis. This step ensures that the vascular region of interest is consistently defined based on the peak tracer activity, which is indicative of the vascular compartment, thereby improving the accuracy of the transfer function coefficient Kf estimation for each kidney.

The step (e) may comprise the following substeps:
(e.i) normalizing the global time sequence,
(e.ii) calculating an average image of images of said global time sequence,
(e.iii) realizing a 2D-convolution on said average image, to obtain a feature map,
(e.iv) identifying on said feature map, for each kidney, a distinct area with a high intensity, corresponding to the position of the kidney under consideration,
(e.v) for each area, cropping the global time sequence images on the area, to obtain a local time sequence of images of one kidney resulting in obtaining, for each kidney, a time sequence of a plurality of images comprising the kidney from said scintigraphy.

Normalizing refers to the process of adjusting measurements of varying scales to a common scale, often to account for noise or to standardize data.

2D-convolution is a mathematical operation used to apply a filter to an image. This involves taking a kernel (a small matrix of numbers) and moving it across the image, applying a mathematical operation (such as summing the product of the kernel values with the image pixel values) at each position to produce a new image. Said kernel may be a 5x5 pixels square kernel.

A feature map typically refers to a processed version of an image or a set of images where specific features or patterns (such as regions of interest, edges, or textures) are highlighted or extracted for further analysis.

Cropping refers to the process of trimming or cutting out a specific area from an image to focus on a particular subject, in this case, each kidney. The cropped images may be centered on each kidney and may be used as a prerequisite for the Patlak calculation, where a more precise contouring of the kidney may be performed to obtain regions of interest that contain all kidney activity and the minimum of extra-renal activity.

Performing a 2D-convolution on the average image to obtain a feature map allows for the enhancement of areas with high tracer activity. This step accentuates the features of interest, such as the kidneys, by emphasizing the boundaries and intensities of these regions, which aids in the subsequent identification and isolation of the kidneys for individual analysis.

Identifying distinct areas with high intensity for each kidney on the feature map enables the precise localization of the kidneys within the scintigraphy images.

Cropping the global time sequence images on the identified areas to obtain a local time sequence of images for each kidney results in a focused dataset that contains only the relevant kidney information. This targeted approach reduces computational complexity and improves the accuracy of the subsequent steps in the method, such as calculating renal tracer activity and estimating split renal function.

Identifying a distinct area with a high intensity may comprise repositioning said area on the closest bean-like shape of said image by:
(e.iv.1) for each of a plurality of diagonal lines, calculating the mean intensity of the pixels composing it, said diagonal lines having different angles in the area under consideration,
(e.iv.2) calculating a weighted center of pixels composing the diagonal line with the highest intensity mean and,
(e.iv.3) centering said area on said center.

The weighted center of pixels, also known as the centroid or center of mass, of a shape or region in an image is calculated by taking the average position of all the pixels in the shape, weighted by their intensity values. For a diagonal line, this involve calculating the average position of the pixels composing the line, taking into account the intensity of each pixel to determine the central point that represents the balance of the pixel intensities along the line.

By calculating the mean intensity of pixels along multiple diagonal lines within a high-intensity area and identifying the diagonal line with the highest mean intensity, the method enables precise localization of the kidney's position within the image. This approach ensures that the region of interest is accurately centered on the kidney.

The repositioning of the high-intensity area to align with the closest bean-like shape, which is characteristic of the kidney's anatomy, enhances the accuracy of the kidney region of interest definition.

The step (f) may comprise the following substeps:
(f.i) from the local time sequence, calculating an average starting image with the first n images of said parenchymal phase, n being a predefined number,
(f.ii) filter said average starting image with an adaptative gaussian threshold with a predefined block size and a predefined constant value,
(f.iii) extend the resulting area outwardly of a predefined number of pixels preferably equal to 1.

An adaptive Gaussian threshold is a method used for image processing where the threshold value for each pixel is determined based on a weighted sum of its neighboring pixels within a specified window, such as a 3x3 neighborhood, minus a constant, for example a constant having a value of 1. This sum is then cross-correlated with a Gaussian window, and pixels not meeting the threshold criteria (e.g., value < pixel value - 1 standard deviation) are excluded. This technique may be applied to enhance the contouring of kidney areas in dynamic renal scintigraphy images by including all relevant kidney activity while minimizing the inclusion of extra-renal activity.

By calculating an average starting image from the first n images of the parenchymal phase, the method enhances the signal-to-noise ratio, providing a clearer representation of the kidney's uptake of the tracer. This facilitates more accurate identification and delineation of the kidney region of interest for subsequent analysis.

The number n of images may be comprised between 9 and 17.

The application of an adaptive gaussian threshold filter with predefined block size and constant value to the average starting image allows for dynamic adjustment to local variations in image intensity. This results in a more precise definition of the kidney region of interest by including pixels that are representative of renal activity while excluding extraneous signals.

Extending the resulting area outwardly by a predefined number of pixels, preferably equal to 1, ensures that the entire kidney region, including any peripheral activity that may be relevant to the function of the kidney, is captured. This extension minimizes the risk of excluding relevant tracer activity that could impact the accuracy of the split renal function estimation.

The step (h) may comprise the following substeps:
(h.i) from the global time sequence, calculating an average ending image with the last m images, m being a predefined number,
(h.ii) determining a vesical region of interest from the average ending image,
(h.iii) detecting a first time of an activity in the vesical region of interest, that time being said end time.

The vesical region refers to the area of the bladder. In the context of dynamic renal scintigraphy, the vesical region is significant as the appearance of vesical activity indicates that the radiotracer has left the kidney regions, which is a critical point for determining when to stop the Patlak-Rutland computation of separated kidney function (SKF).

The number m of images may be comprised between 5 et 15.

Determining a vesical region of interest from the average ending image allows for the precise localization of the bladder activity.

Detecting the first time of activity in the vesical region of interest serves as an indicator of when the radiotracer has left the kidney regions. This information is useful for accurately determining the end time of renal activity and ensuring that the calculation of the transfer function coefficient Kf is based on the correct interval.

The step (h.ii) may comprise the following substeps:
(h.ii.1) determining, in the area below the kidney region of interest, a set of vesical region of interest candidate,
(h.ii.2) selecting from said vesical region of interest candidates the upmost region as the vesical region of interest.

By determining a set of vesical region of interest candidates below the kidney region of interest, the method enhances the accuracy of identifying the vesical region, in order to determine the end time of renal activity. This step ensures that the vesical activity is correctly captured, which permits the accurate computation of the split renal function using the Patlak-Rutland method.

Selecting the upmost region from the vesical region of interest candidates as the vesical region of interest allows the method to differentiate between potential urine zones and the actual bladder activity. This distinction is particularly important in cases where micturition occurs during the scintigraphy, especially in infants, to prevent incorrect identification of vesical activity and to ensure the integrity of the split renal function calculation.

The step (h) may comprise the following substeps:
(h.i) detecting a decrease of the activity in the kidney region of interest during p consecutive images, p being a predefined number,
(h.ii) defining the end time as the time corresponding to the first image of said p consecutive images.

By detecting a decrease of activity in the kidney region of interest during a predefined number of consecutive images, the method provides a reliable indication of when the tracer begins to leave the kidney region. This allows for the accurate determination of the end time for the Patlak-Rutland computation.

Defining the end time as the time corresponding to the first image of the predefined number of consecutive images with decreased activity simplifies the process of identifying the appropriate time frame for the Patlak-Rutland computation. This approach reduces the complexity of the analysis and minimizes the potential for human error in the interpretation of scintigraphy data, thereby enhancing the robustness and reproducibility of the method for estimating split renal function in clinical settings.

The step (i) may estimate a ratio between transfer function coefficients Kf of each kidney by calculating a ratio over time, from the start time to the end time, between the renal activity and the vascular activity as an affine function of the transfer function coefficient Kf. More specifically, this ratio is obtained by comparing the slope of the lines defined by the affine functions. This affine function is intrinsic to the Patlak method. It is precisely the existence of a linear relationship during the defined calculation time that allows Kf to be compared between each kidney by comparing the slope of the straight lines formed.

An affine function is a mathematical expression representing a relationship that is linear in nature but does not necessarily pass through the origin. The affine function in this context would typically take the form of y = ax + b, where 'a' represents the slope, 'b' is the y-intercept, and 'x' and 'y' are variables representing quantities.

The use of an affine function of the transfer function coefficient Kf facilitates the derivation of a linear relationship that simplifies the computation of the split renal function, enhancing the reliability and precision of the diagnostic information obtained from the scintigraphy.

The step (j) may estimate the separated kidney function as the ratio of both transfer function coefficients Kf.

By estimating the separated kidney function as the ratio of both transfer function coefficients Kf, the method provides a quantitative assessment of the relative function of each kidney. This allows for a direct comparison of renal function between the two kidneys, in order to diagnose and monitor renal diseases, planning surgeries, and evaluating the effects of therapeutic interventions.

The present disclosure also relates to a computer-readable non-transient recording medium on which a software is registered to implement the above-mentioned method when the software is executed by a processor.

The present disclosure also relates to a computer program comprising instructions for implementing the above-mentioned method, when this program is executed by a processor.

The present disclosure also relates to a calculating unit comprising:
- an input interface to receive at least images of a dynamic renal scintigraphy and a starting time of a parenchymal phase on said scintigraphy,
- a memory comprising at least the instructions of an above-mentioned computer program,
- a processor having access to said memory to read said instruction and executing the above-mentioned method,
- an output interface to output a separated kidney function value.

### Brief Description of Drawings

Other features, details and advantages will be shown in the following detailed description and on the figures, on which:
[Fig. 1] is a schematic diagram illustrating a computer device according to the present document,
[Fig. 2] is a schematic diagram illustrating the method according to the present document,
[Fig. 3] is an example of a dynamic renal scintigraphy image.

### Description of Embodiments

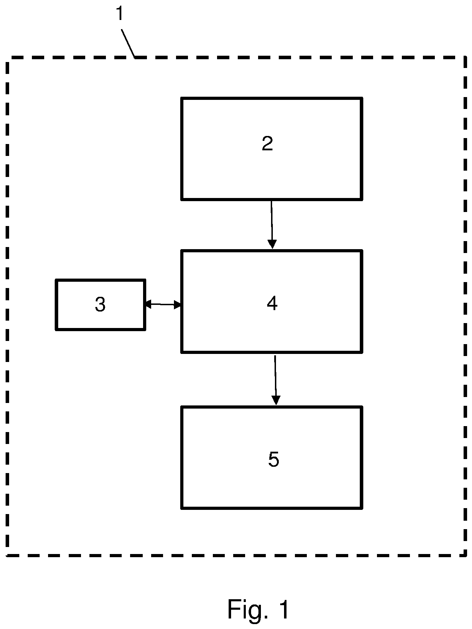

Figure 1 shows a computer device 1 comprising:
- an input interface 2 to receive said at least one time series signals from an actual sensor,
- a memory 3 for storing at least instructions of a computer program and executing a method according to the present document, described below,
- a processor 4 accessing to the memory 3 for reading and executing the aforesaid instructions,
- an output interface 5 to provide an information concerning the evolution of said stationarity.

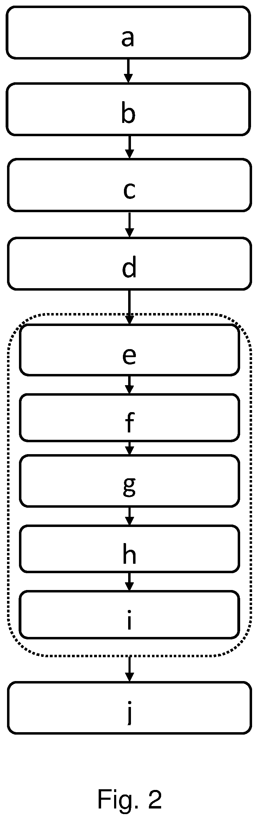

The method according to the present document is shown in Figure 2. Said method aims to estimate, on a dynamic renal scintigraphy of both kidneys of a patient, a split renal function for a transfer renal function of a tracer in the kidneys. Said dynamic renal scintigraphy comprises a series of images taken over a global time sequence.

Said method comprises a first step in which at least one series of images is obtained during a global time sequence of a dynamic renal scintigraphy, at least one image comprising both kidneys (step (a)).

Then, the starting time of a parenchymal phase of the global time sequence is obtained from said dynamic renal scintigraphy (step (b)).

Next, a vascular region of interest is defined on the images of the global time sequence (step (c)).

To this aim, during step (c), a preliminary average image is calculated from the images of the global time sequence taken before the starting time. If there are no images before the starting time, the average image is simply the first image of the global time sequence (step (c.i)).

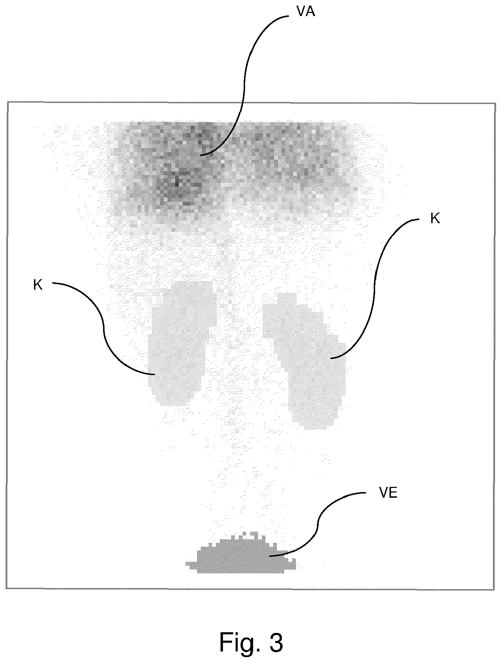

Then an upper area with the highest intensity is identified on said preliminary average image (step (c.ii)) and said vascular region of interest (VA in figure 3) is defined on said upper area (step (c.iii)).

Afterward, the tracer activity in the vascular region of interest is calculated on the images, referred to as vascular tracer activity (step (d)).

For each kidney, a local time sequence of a plurality of images comprising the kidney is obtained from the series of images taken during the global time sequence (step (e)).

To this aim, during step (e), the global time sequence is firstly normalized (step (e.i)). Then, an average image is calculated from the images of the global time sequence (step (e.ii)).

Afterward, a 2D convolution is performed on the average image to generate a feature map (step (e.iii)).

On this feature map, a distinct area with high intensity is identified for each kidney (K in figure 3), corresponding to the location of said kidney (step (e.iv)). Such identification is performed by repositioning said area on the closest bean-like shape of said image. To this end, for each of a plurality of diagonal lines, the mean intensity of the pixels composing said diagonal is calculated (step (e.iv.1)), said diagonal lines having different angles in the area under consideration. Then a weighted center of pixels composing the diagonal line with the highest intensity mean is calculated (step (e.iv.2)) and, said area is centered on said center (step (e.iv.3)).

Finally, for each identified area, the global time sequence images are cropped to obtain a local time sequence of kidney images, resulting in a time sequence of multiple images for each kidney derived from the scintigraphy (step (e.v)).

Subsequently, a kidney region of interest is defined on the images of the local time sequence (step (f)).

To this aim, from the local time sequence, an average starting image is calculated using the first n images of the parenchymal phase, where n is a predefined number (step (f.i)).

Next, this average starting image is filtered using an adaptive Gaussian threshold with a predefined block size and constant value (step (f.ii)).

Lastly, the resulting area is extended outward by a predefined number of pixels, preferably one (step (f.iii)).

Then, the tracer activity in the kidney region of interest for each image of the local time sequence, called renal tracer activity, is calculated (step (g)).

Following this, an end time of the renal activity in the kidney region of interest is determined ((step (h)).

To this aim, from the global time sequence, an average ending image is calculated using the last m images, where m is a predefined number (step (h.i)).

Then, a vesical region of interest is determined from the average ending image (step (h.ii)). To that end, in the area below the kidney region of interest, a set of vesical region of interest candidates is identified (step (h.ii.1)) and, from these vesical region of interest candidates, the uppermost region is selected as the vesical region of interest (step (h.ii.2), see VE in Figure 3).

Finally, the first time of activity in the vesical region of interest is detected, with this time being the end time (step (h.iii)).

Alternatively, the step (h) is performed by detecting a decrease of the activity in the kidney region of interest during p consecutive images, p being a predefined number, and defining the end time as the time corresponding to the first image of said p consecutive images.

A ratio between transfer function coefficients Kf of each kidney is then estimated from the renal and vascular activities in the kidney region of interest after the starting time (step (i)), by comparing the slopes of the straight lines of the affine function of each kidney, proportional to the Kf of the concerned kidney.

Lastly, the split renal function is estimated from said ratio (step (j)).

## Claims

1. A method implemented by computer means for estimating, on a dynamic renal scintigraphy of both kidneys of a patient, a split renal function for a transfer renal function of a tracer in the kidneys, said dynamic renal scintigraphy comprising a series of images taken over a global time sequence, said method comprising the following steps:
(a) obtaining said series of images during a global time sequence, at least one image comprising both kidneys,
(b) obtaining a starting time of a parenchymal phase of the global time sequence from said dynamic renal scintigraphy,
(c) defining a vascular region of interest on said images of the global time sequence,
(d) calculating a tracer activity in the vascular region of interest on said images, called vascular tracer activity;
wherein, for each kidney, the method comprises:
(e) obtaining a local time sequence of a plurality of images comprising the kidney from said series of images obtained during the global time sequence,
(f) defining a kidney region of interest on said images of the local time sequence,
(g) calculating a tracer activity in the kidney region of interest for each image of said local time sequence, called renal tracer activity,
(h) determining an end time of the renal activity in the kidney region of interest,
wherein the method further comprises the following step:
(i) estimating a ratio between transfer function coefficients Kf of each kidney at least from the renal and vascular activities in the kidney regions of interest after said starting time;
(j) estimating the split renal function at least from said ratio between the transfer function coefficients.

2. The method of claim 1 wherein the step (c) comprises the following substeps:
(c.i) calculating a preliminary average image of images of said global time sequence before the starting time, said average image being the first image of said global time sequence if none before,
(c.ii) identifying on said preliminary average image an upper area with the highest intensity,
(c.iii) defining said vascular region of interest on said upper area.

3. The method of claim 1 or 2 wherein the step (e) comprises the following substeps:normalizing the global time sequence,
(e.ii) calculating an average image of images of said global time sequence,
(e.iii) realizing a 2D-convolution on said average image, to obtain a feature map,
(e.iv) identifying on said feature map, for each kidney, a distinct area with a high intensity, corresponding to the position of the kidney under consideration,
(e.v) for each area, cropping the global time sequence images on the area, to obtain a local time sequence of images of one kidney resulting in obtaining, for each kidney, a time sequence of a plurality of images comprising the kidney from said scintigraphy.

4. The method of claim 3, wherein identifying a distinct area with a high intensity comprises repositioning said area on the closest bean-like shape of said image by:
(e.iv.1) for each of a plurality of diagonal lines, calculating the mean intensity of the pixels composing it, said diagonal lines having different angles in the area under consideration,
(e.iv.2) calculating a weighted center of pixels composing the diagonal line with the highest intensity mean and,
(e.iv.3) centering said area on said center.

5. The method according to any preceding claim wherein the step (f) comprises the following substeps:(f.i) from the local time sequence, calculating an average starting image with the first n images of said parenchymal phase, n being a predefined number,
(f.ii) filter said average starting image with an adaptative gaussian threshold with a predefined block size and a predefined constant value,
(f.iii) extend the resulting area outwardly of a predefined number of pixels preferably equal to 1.

6. The method according to any preceding claim wherein the step (h) comprises the following substeps:
from the global time sequence, calculating an average ending image with the last m images, m being a predefined number,
(h.ii) determining a vesical region of interest from the average ending image,
(h.iii) detecting a first time of an activity in the vesical region of interest, that time being said end time.

7. The method according to the preceding claim wherein the step (h.ii) comprises the following substeps:
determining, in the area below the kidney region of interest, a set of vesical region of interest candidate,
(h.ii.2) selecting from said vesical region of interest candidates the upmost region as the vesical region of interest.

8. The method according to one of the preceding claims 1 to 5 wherein the step (h) comprises the following substeps:
(h.i) detecting a decrease of the activity in the kidney region of interest during p consecutive images, p being a predefined number,
(h.ii) defining the end time as the time corresponding to the first image of said p consecutive images.

9. The method according to any preceding claim wherein the step (i) estimates a ratio between the transfer function coefficients Kf of each kidney by calculating a ratio over time, from the start time to the end time, between the renal activity and the vascular activity as an affine function of the transfer function coefficient Kf.

10. The method according to any preceding claim wherein the step (j) estimates the separated kidney function as the ratio of both transfer function coefficients Kf.

11. Computer-readable non-transient recording medium on which a software is registered to implement a method according to any of the preceding claims when the software is executed by a processor.

12. Computer program comprising instructions for implementing the method according to one of the preceding claims, when this program is executed by a processor.

13. Calculating unit comprising:
- an input interface to receive at least images of a dynamic renal scintigraphy and a starting time of a parenchymal phase on said scintigraphy,
- a memory comprising at least the instructions of a computer program according to the preceding claim,
- a processor having access to said memory to read said instruction and executing the method according to one of the claims 1 to 10,
- an output interface to output a separated kidney function value.
